# EUROPEAN PATENT SPECIFICATION

(11) **EP 4 316 415 B1**
(45) Date of publication and mention of the grant of the patent: **01.07.2026**
(21) Application number: 21794944.5
(22) Date of filing: 07.09.2021
(51) Int. Cl.: A61C 9/00, A61B 1/24, A61B 5/00, A61C 11/00

(54) **INTRAORAL SCANNER**
INTRAORALER SCANNER
NUMÉRISEUR INTRA-ORAL

(30) Priority: 26.03.2021 PT 2021117148
(43) Date of publication of application: 07.02.2024
(73) Proprietor: Santos Redinha, Luís António Dos, 2770-174 Paço de Arcos (PT)
(72) Inventor: Santos Redinha, Luís António Dos, 2770-174 Paço de Arcos (PT)
(74) Representative: Pereira da Cruz, Joao
(86) International application number: PCT/IB2021/058124
(87) International publication number: WO 2022/200849

(56) References cited:
- DE-U1- 202020 003 736
- US-A- 4 908 949
- US-A- 5 069 619
- US-A1- 2013 218 530
- US-A1- 2014 302 452
- US-A1- 2016 030 144
- US-A1- 2019 298 502
- US-B1- 10 810 738
- US-B1- 6 261 248
- US-B2- 11 033 367

## Description

### TECHNICAL FIELD OF THE INVENTION

The present invention falls within the field of medical devices, specifically medical devices for use in dentistry, in particular intraoral scanning devices, even more particularly intraoral scanners for acquisition of the natural head position, three-dimensional orientation of the occlusal plane position and facial aesthetic references.

### TECHNICAL SCOPE OF THE INVENTION

The use of Intra Oral Scanners (IOS) is a growing and irreversible trend in the various fields of dental medicine. There is already a full digital flow in the areas of oral rehabilitation and orthodontics, and this will gradually lead to the disappearance of analogue workflows. However, the information collected by the current Intraoral Scanners is not sufficient for complex cases of oral rehabilitation and clear aligner systems. In these cases, the additional information provided by the current analogue systems, such as the use of the Face Arch, and other analogue systems such as *Plane System by Udo Plaster, Kois DentoFacial Analyser, Ditramax, Aesthetic Cross Reference,* etc....

The data collected by these complementary analogue systems may relate to functional elements, which focus on the functional relation existing between the spatial position of the jaws and the Temporomandibular Joint (TMJ) or elements such as:
- three-dimensional (3D) position of the upper jaw and orientation of the occlusal plane in relation to the face;
- interpupillary line and natural head position;
Some systems have attempted to integrate in the same protocol the two components although with only relative success, as explained below.

The subsequent digitalisation of the data obtained by these systems currently allows the achievement of digital workflows.

However, for the effective creation of a fully digital workflow, complementary tools/protocols similar to the analogue ones previously mentioned are needed, since the digital prints are currently transferred to the screen in a horizontal position that does not represent the 3D spatial position present in the patient and the positioning when mounting in the digital articulator is done based upon mean values or performed manually by the operator, trying to reproduce the 3D spatial position of the maxilla present in the patient through visual information and, for instance, comparatively, an example of this is the use of photographs.

### STATE OF THE ART OF THE INVENTION

Although there are currently some systems available on the market that attempt to provide this information digitally, such as the face scanner products *Sicat by Sirona* and *JMA System by Zebris,* their cost, complexity and time-consuming use make them expensive and inappropriate for dentists.

The incorporation of a virtual face bow (VFB) in the scanning protocol and assembly in virtual articulator for IOS has already been proposed, by the inventor of this patent application, in publication WO2016/055932A1, which discloses a correct acquisition of the functional components. However, due to its characteristics, the incorporation of a virtual face bow does not allow, in some cases, the correct determination of vital elements for the treatment planning, since it has limitations in determining the natural head position (NHP) and also in the three-dimensional spatial orientation of the maxilla regarding prosthodontic and orthodontic aesthetic determinants.

Document US20140302452A1 discloses a dental camera system for creating an image of an object that has a dental camera, which comprises a housing and optics arranged therein together with an image sensor. The dental camera system further comprises an evaluation unit which cooperates with the image sensor to capture the object. To achieve a dental camera system whereof the dental camera performs using optics without movable or controllable optical elements, it is provided for the optics to be plenoptic optics which are designed to determine a 4D light field when capturing the object and for the evaluation unit to be designed to calculate at least one representation of the captured object from the 4D light field.

Document US20160030144A1 discloses a digital face bow system for capturing a patient's dento-facial characteristics by acquiring and registering data defining the tilt or slant of the occlusal or incisal plane of a patient's teeth in three planes of space in relation to the cranium, head and/or face. The system includes a substrate adapted to support bite registration material, and a digital multiple axis inclinometer coupled to the substrate for registering the orientation of the occlusal or incisal plane, where the midline of the patient's horizontal-facial plane is registered. An adjustable mounting platform is adapted to receive the substrate and replicate the inclination registered by the digital multiple axis inclinometer.

### SUMMARY OF THE INVENTION

The present invention relates to a method for facial analysis, acquisition of the natural head position and facial aesthetic references and is defined in the appended claims.

### DESCRIPTION OF THE FIGURES

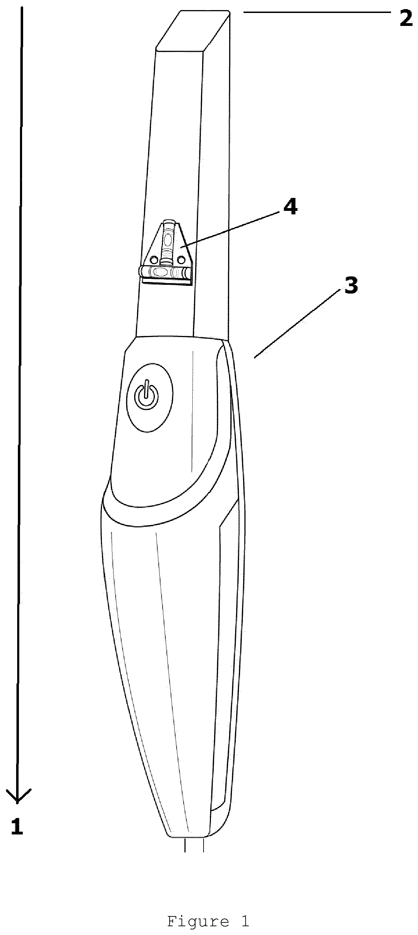

Figure 1 - Representation of the present invention, with orientation of the information collection system (2) of the intraoral scanner (3), in relation to the object to be scanned in a position exactly orthogonal to the vertical plane defined by a plumb line (1), by checking the positioning of the level (4), in this representation a double bubble level.

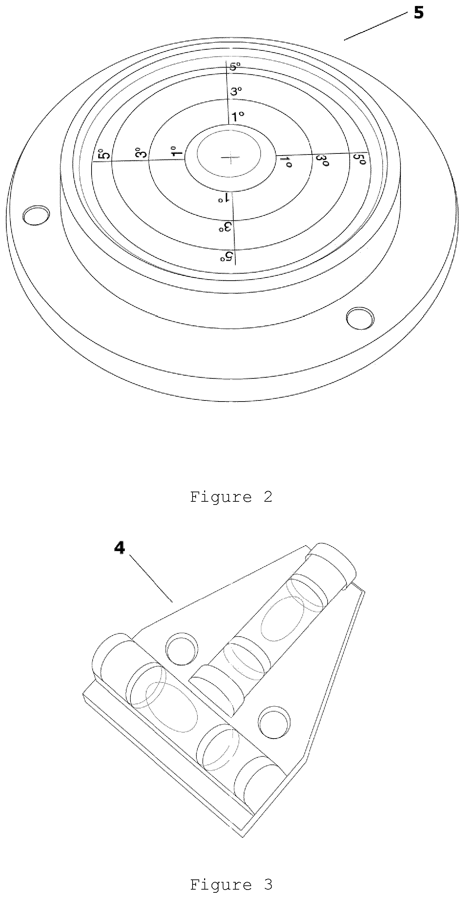

Figure 2 - Representation of the present invention in a preferred embodiment in which the bubble level is an analogue double bubble level (4).
Figure 3 - Representation of the present invention in a preferred embodiment, in which the bubble level is an analogue circular bubble level (5).

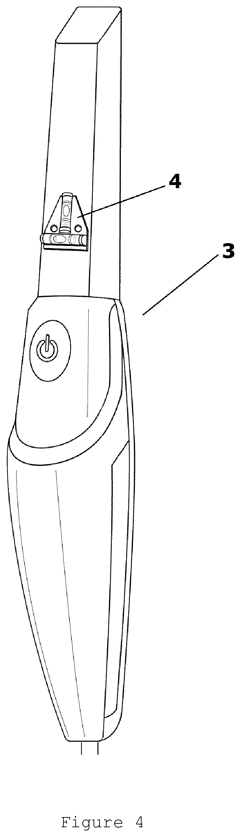

Figure 4 - Representation of the intraoral scanner (3), with a double bubble level (4).

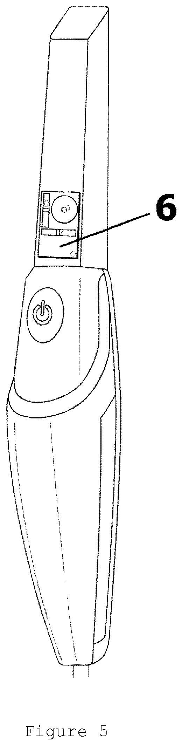

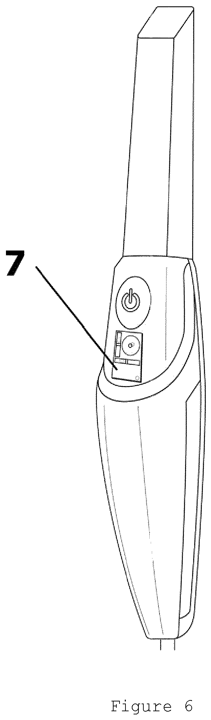

Figures 5 and 6 - Representations of the present invention in a preferred embodiment, in which the level is digital (6), comprising an electronic display (7) on the body of the intraoral scanner, in various positions provided that they allow its reading.

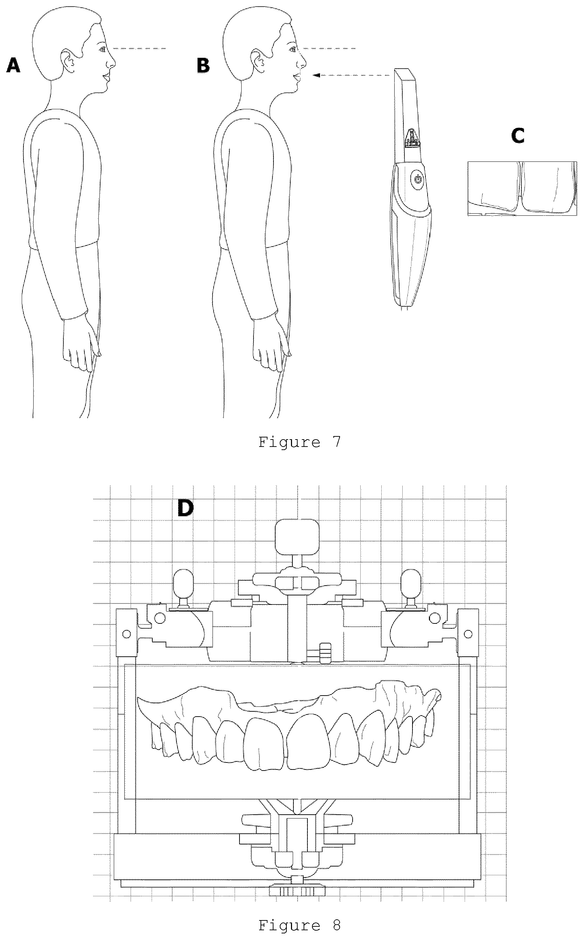

Figure 7 - Representation of the new acquisition method and its steps A, B and C, described herein.
Figure 8 - Representation of step D of the new method of transfer to the Virtual Articulator.

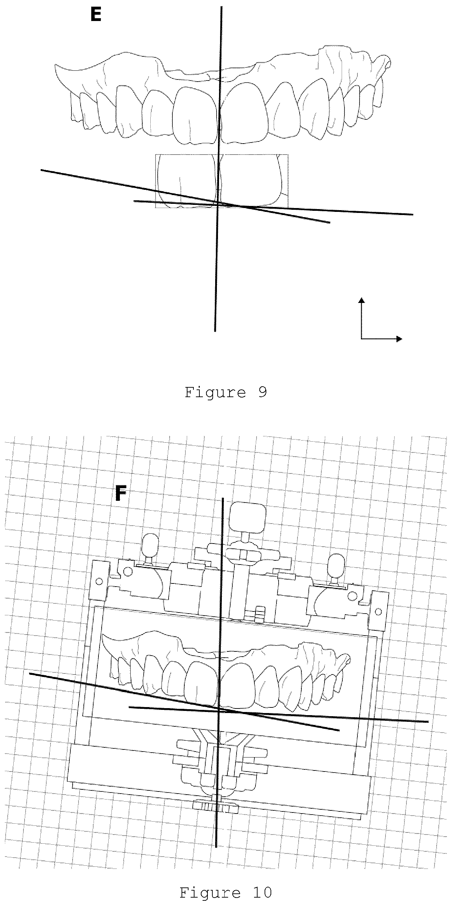

Figure 9 - Representation of step E of the new method of transfer to the Virtual Articulator.
Figure 10 - Representation of step F of the new method of transfer to the Virtual Articulator.

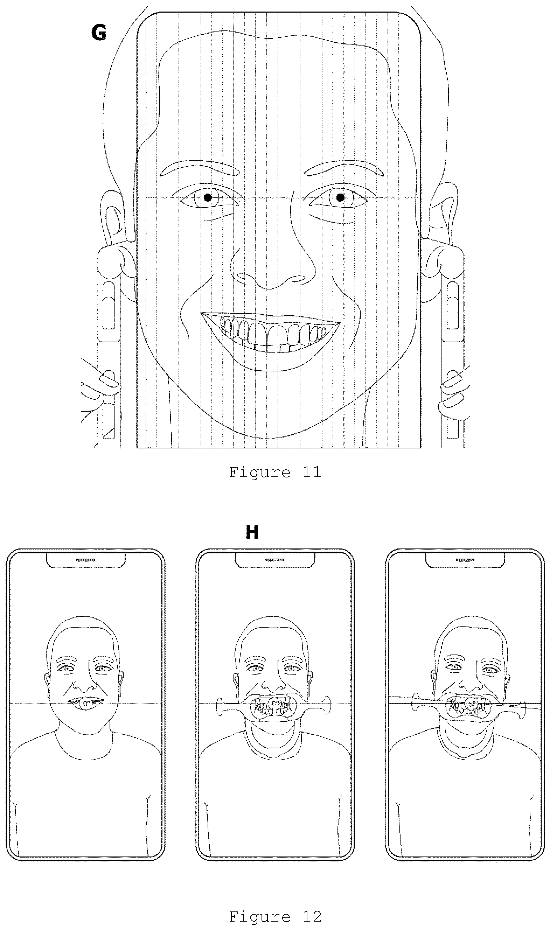

Figure 11 - Representation of step G of the new method of transfer to the Virtual Articulator.

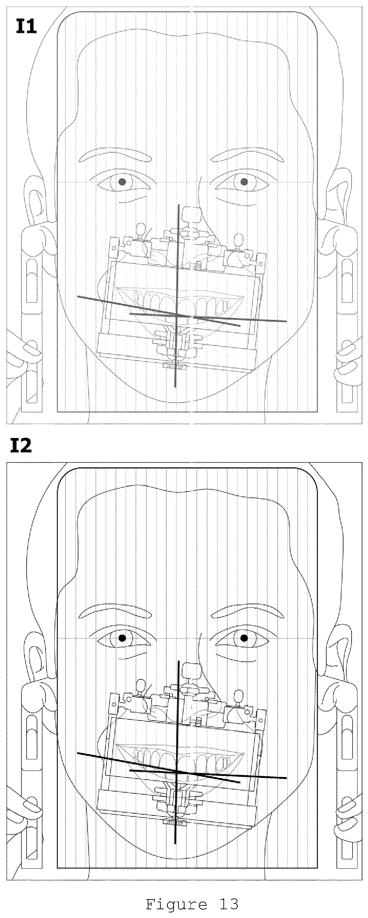

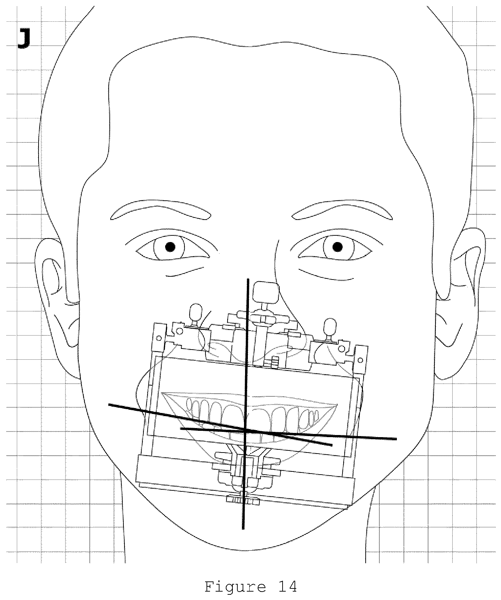

Figures 12, 13 and 14 - Representation of steps H, I (I1, I2) and J of the new method of transfer to the Virtual Articulator H.

### DESCRIPTION OF THE INVENTION

The present disclosure relates to an intraoral scanner (3) which, in particular, allows the acquisition of the natural head position (NHP), the three-dimensional orientation of the occlusal plane position and the facial aesthetic references, comprising a bubble level, which may be analogue (4) (5) or digital (6), enabling the orientation of the information collection system (2) of the intraoral scanner (3) in relation to the object to be scanned in a position exactly orthogonal to the vertical plane defined by a plumb line (1).

The bubble level is inserted or integrated into the intraoral scanner by means of a mechanical or magnetic retention fitting.

In a preferred embodiment, the bubble level is either an analogue double bubble (4) or an analogue circular bubble (5) or a digital one (6). An analogue level (4) (5) is always guaranteed to function, a digital level (6) must be powered by a power source, e.g. batteries. However, this type of digital level (6) has higher levels of precision intended for works which require it.

In a preferred embodiment, and in case the level is digital (6), besides the fitting being possible by mechanical or magnetic retention, the fitting may also be of the camera "flash" type that allows for power supply.

In a preferred embodiment, and in case the level is digital (6), it can comprise an electronic display (7) on the body of the intraoral scanner, in various positions as long as they allow its reading.

The present invention implements a new method for acquiring the natural head position, the three-dimensional orientation of the occlusal plane position and the facial aesthetic references, which comprises the following steps:
A. after scanning the maxillary arches, with or without performing the protocol for incorporating a virtual face bow, the patient stands in a relaxed position and looks straight ahead at a fixed point on the horizon, keeping that position;
B. the patient lifts the upper lip in order to expose the upper central incisors or he/she can use a (spring) lip retractor for the same purpose;
C. the intraoral scanner body (3) is levelled so that the acquisition beam (2) is orthogonal to the vertical plane of the plumb line (1), then the maxillary central incisors are scanned in such a way as to acquire the interincisor midline and the maximum extension of the incisor edges allowed by the objective lens of the intraoral scanner (3);
D. mounting in the digital articulator, using mean values or through the virtual face bow protocol;
E. superimposing the image of the upper incisors obtained in the patient's natural head position onto the upper model mounted on the virtual articulator;
F. three-dimensional orientation of the articulator;
G. the operator works from a real "line of sight" perspective and maintains the functional relation of the jaws with the condyles of the temporomandibular joint (TMJ);
H. acquisition of extra-oral photograph of the patient, which can be done using a mobile device with an application like "Spirit Level Camera+", that through the inclinometer level allows the incorporation of the natural position of the head, as well as the collection of information about the facial aesthetic determinants;
I. superimposition of extra oral photographs taken with the patient in the natural head position and which can be executed with the use of a camera with inclinometer adjusted to the vertical of the plumb line (1); or
J. superimposing a face scanner.

This new method of Facial Aesthetic References Acquisition allows the placement of the facial aesthetic determinants in an exact position in relation to the occlusal plane of the patient, thus providing a facial analysis with a high level of accuracy.

As will be evident to a person skilled in the art, the present invention should not be limited to the embodiments described herein, with a number of changes being possible, which remain within the scope of this invention.

Of course, the preferred embodiments above described are combinable in the different possible forms, the repetition of all such combinations being herein avoided.

## Claims

1. A method for facial analysis, acquisition of the natural head position and facial aesthetic references comprising the following steps:
A. scanning a maxillary arch of a patient with an intra-oral scanner, with or without performing a virtual facebow protocol;
B. level an intraoral scanner (3) comprising an information collection system (2) **characterized in that** it comprises a bubble level (4) (5) (6) integrated into a surface of the intraoral scanner by means of a mechanical or magnetic retention fitting, in front of the patient with a head in its natural position and exposing the upper central incisors, so that an acquisition beam of the information collection system (2) is orthogonal to a vertical plane defined by a plumb line (1);
C. scanning maxillary central incisors in such a way as to acquire an interincisor midline and a maximum extension of the incisor edges allowed by an objective lens of the intraoral scanner (3);
D. mounting the result of the scanning obtained in step A in a virtual articulator, using mean values or through a virtual face bow protocol;
E. superimposing an image of the upper incisors obtained in step C onto the model of maxillary arch obtained in step A mounted on the virtual articulator;
F. acquisition of extra-oral photograph of the frontal part of the head of the patient in its natural position, and collect from the photograph information about facial aesthetic determinants, **characterized in that** the photographs are executed using a camera with an inclinometer set to the vertical of the plumb line (1); and/or a mobile device with a "Spirit Level Camera+" type application; and
G. superimposition of i) extra oral photographs of the head of the patient obtained in the previous step; or ii) a scan of the face of the patient, on the result of step D.

2. Method according to claim 1, **characterized in that** the bubble level (4) is an analogue double bubble level.

3. Method according to claim 1, **characterized in that** the bubble level (5) is an analogue circular bubble level.

4. Method according to claim 1, **characterized in that** the bubble level (6) is a digital bubble level.

5. Method according to claim 1, **characterized in that** the fitting is of mechanical or magnetic retention, or of the camera "flash" type.

6. Method according to claims 1 and 4, **characterized in that** the level (6) comprises an electronic display (7) in the body of the intraoral scanner (3).

## Patentansprüche

1. Verfahren zur Gesichtsanalyse, zur Erfassung der natürlichen Kopfposition und ästhetischer Referenzpunkte im Gesicht, das die folgenden Schritte umfasst:
A. Scannen eines Oberkieferbogens eines Patienten mit einem Intraoralscanner, mit oder ohne Durchführung eines virtuellen Gesichtsbogenprotokolls;
B. Ausrichten eines intraoralen Scanners (3), der ein Informationserfassungssystem (2) umfasst, **dadurch gekennzeichnet, dass** er eine Wasserwaage (4) (5) (6), die mittels einer mechanischen oder magnetischen Halterung in eine Oberfläche des Intraoralscanners integriert ist, vor dem Patienten mit dem Kopf in seiner natürlichen Position und unter Freilegung der oberen mittleren Schneidezähne, so dass ein Erfassungsstrahl des Informationserfassungssystems (2) orthogonal zu einer durch ein Lotstrang (1) definierten vertikalen Ebene verläuft;
C. Scannen der oberen mittleren Schneidezähne derart, dass eine interinzisale Mittellinie und eine maximale Ausdehnung der Schneidezahnkanten erfasst werden, die durch ein Objektiv des Intraoralscanners (3) ermöglicht wird;
D. Einpassen des in Schritt A erhaltenen Scanergebnisses in einen virtuellen Artikulator unter Verwendung von Mittelwerten oder mittels eines virtuellen Gesichtsbogenprotokolls;
E. Überlagerung eines in Schritt C erhaltenen Bildes der oberen Schneidezähne auf das in Schritt A erhaltene Modell des Oberkieferbogens, das auf dem virtuellen Artikulator montiert ist;
F. Aufnahme eines extraoralen Fotos des vorderen Kopfbereichs des Patienten in seiner natürlichen Position und Erfassung von Informationen über ästhetische Gesichtsmerkmale aus dem Foto, **dadurch gekennzeichnet, dass** die Fotos mit einer Kamera aufgenommen werden, deren Neigungsmesser auf die Senkrechte des Lotstrangs (1) eingestellt ist; und/oder mit einem mobilen Gerät mit einer Anwendung vom Typ "Spirit Level Camera+"; und
G. Überlagerung von i) im vorherigen Schritt aufgenommenen extraoralen Fotos des Kopfes des Patienten; oder ii) einem Scan des Gesichts des Patienten mit dem Ergebnis von Schritt D.

2. Verfahren nach Anspruch 1, **dadurch gekennzeichnet, dass** die Wasserwaage (4) eine analoge Doppelwasserwaage ist.

3. Verfahren nach Anspruch 1, **dadurch gekennzeichnet, dass** die Wasserwaage (5) eine analoge Rundwasserwaage ist.

4. Verfahren nach Anspruch 1, **dadurch gekennzeichnet, dass** die Wasserwaage (6) eine digitale Wasserwaage ist.

5. Verfahren nach Anspruch 1, **dadurch gekennzeichnet, dass** die Halterung mechanisch oder magnetisch ist oder vom Typ eines Kamerablitzes ist.

6. Verfahren nach den Ansprüchen 1 und 4, **dadurch gekennzeichnet, dass** die Wasserwaage (6) eine elektronische Anzeige (7) im Gehäuse des Intraoralscanners (3) umfasst.

## Revendications

1. Procédé d'analyse faciale, d'acquisition de la position naturelle de la tête et d'identification de repères esthétiques du visage, comprenant les étapes suivantes:
A. numérisation de l'arcade maxillaire d'un patient à l'aide d'un scanner intra-oral, avec ou sans mise en œuvre d'un protocole d'arc facial virtuel;
B. mise à niveau d'un scanner intra-oral (3) comprenant un système de collecte d'informations (2), **caractérisé en ce qu'**il comprend un niveau à bulle (4) (5) (6) intégré dans une surface du scanner intra-oral au moyen d'un dispositif de retenue mécanique ou magnétique, en face du patient, la tête en position naturelle et exposant les incisives centrales supérieures, de sorte qu'un faisceau d'acquisition du système de collecte d'informations (2) soit orthogonal à un plan vertical défini par un fil à plomb (1) ;
C. numérisation des incisives centrales maxillaires de manière à acquérir une ligne médiane interincisive ainsi qu'une extension maximale des bords incisifs permise par l'objectif du scanner intra-oral (3) ;
D. montage du résultat de la numérisation obtenu à l'étape A dans un articulateur virtuel, au moyen de valeurs moyennes ou par mise en œuvre d'un protocole d'arc facial virtuel ;
E. superposition d'une image des incisives supérieures obtenue à l'étape C sur le modèle de l'arcade maxillaire obtenu à l'étape A monté sur l'articulateur virtuel ;
F. acquisition d'une photographie extra-orale de la partie frontale de la tête du patient en position naturelle, et collecte, à partir de ladite photographie, d'informations relatives aux déterminants esthétiques faciaux, **caractérisée en ce que** les photographies sont réalisées au moyen d'un appareil photographique muni d'un inclinomètre réglé selon la verticale définie par le fil à plomb (1) ; et/ou d'un dispositif mobile équipé d'une application de type "Spirit Level Camera+" ; et
G. superposition i) de photographies extra-orales de la tête du patient obtenues à l'étape précédente; ou ii) d'une numérisation du visage du patient, sur le résultat de l'étape D.

2. Procédé selon la revendication 1, **caractérisé en ce que** le niveau à bulle (4) est un niveau à double bulle analogique.

3. Procédé selon la revendication 1, **caractérisé en ce que** le niveau à bulle (5) est un niveau à bulle circulaire analogique.

4. Procédé selon la revendication 1, **caractérisé en ce que** le niveau à bulle (6) est un niveau à bulle numérique.

5. Procédé selon la revendication 1, **caractérisé en ce que** le dispositif de retenue est de type mécanique ou magnétique, ou de type "flash" d'appareil photographique.

6. Procédé selon les revendications 1 et 4, **caractérisé en ce que** le niveau (6) comprend un dispositif d' affichage électronique (7) dans le corps du scanner intra-oral (3).
